(19) Europäisches Patentamt
European Patent Office
Office européen des brevets

(11) **EP 3 455 179 B1**

(12) **EUROPEAN PATENT SPECIFICATION**

(45) Date of publication and mention
of the grant of the patent:
**08.07.2020 Bulletin 2020/28**

(21) Application number: **17723672.6**

(22) Date of filing: **11.05.2017**

(51) Int Cl.:
***C03C 1/00*** *(2006.01)*    ***C03C 1/02*** *(2006.01)*
***C03B 1/02*** *(2006.01)*    ***C03C 13/06*** *(2006.01)*

(86) International application number:
**PCT/EP2017/061420**

(87) International publication number:
**WO 2017/194726 (16.11.2017 Gazette 2017/46)**

(54) **COMPACTED BODY**

VERDICHTETER KÖRPER

CORPS COMPRIMÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.05.2016 EP 16169635
13.05.2016 EP 16169638
13.05.2016 EP 16169641**

(43) Date of publication of application:
**20.03.2019 Bulletin 2019/12**

(73) Proprietors:
• **Rockwool International A/S
2640 Hedehusene (DK)**
• **Flumroc AG
8890 Flums (CH)**

(72) Inventors:
• **PFIFFNER, Roland
8880 Walenstadt (CH)**
• **CEKO, Ivan
8808 Pfäffikon (CH)**
• **HJELMGAARD, Thomas
3480 Fredensborg (DK)**

(74) Representative: **Letzelter, Felix Phillip
Meissner Bolte Patentanwälte
Rechtsanwälte Partnerschaft mbB
Widenmayerstraße 47
80538 München (DE)**

(56) References cited:
**WO-A1-2010/106181    WO-A1-2010/132641
WO-A1-2012/013780**

**Description**

Field of the Invention

The present invention relates to a compacted body, in particular a briquette, suitable for use as a mineral charge in the production of man-made vitreous fibres (MMVF).

Background of the Invention

Man-made vitreous fibres (MMVF) such as, e.g., glass fibres, ceramic fibres, basalt fibres, slag fibres and stone fibres, may be made by melting a mineral charge in a furnace and fiberising the melt, usually by a centrifugal fiberising process such as, for instance, by a spinning cup process or a cascade rotor process. The MMV fibres produced may form wool products such as glass wool or stone wool.

In some of the furnaces used for MMVF production, there is a large pool of melt and the mineral charge is melted into this pool. Examples are tank and electric furnaces, which can be used for rock fibre production but mostly for glass fibre production.

Another type of furnace which is used for forming the melt for MMVF production, especially of fibres of the types that are referred to as stone, slag and basalt fibres, is a shaft furnace or cupola furnace which contains a self-supporting column of solid coarse mineral and combustion material, and combustion gases permeate through this column so as to heat it and cause melting. The melt drains to the bottom of the column, where a pool of melt is usually formed, and the melt is removed from the base of the furnace. Since the column has to be both self-supporting and permeable it is necessary that the raw material should be relatively coarse and should have considerable strength, despite the high temperatures in the column which may exceed 1000°C.

The raw material can be formed of coarsely crushed, naturally occurring rock and slag or any other type of suitable coarse material, provided this will withstand the pressures and temperatures in the self-supporting column in the shaft furnace. When applying more fine grained raw materials it is known to convert the finer particulate materials such as sands into bonded briquettes for addition to the furnace. These should have sufficient strength and temperature resistance to withstand the conditions in the self-supporting column in the shaft furnace in order that they melt prior to collapsing.

It is necessary for the total charge in the furnace (i.e., lump mineral alone or lump mineral plus briquettes) to provide the composition which is desired for the MMV fibres which are to be made. However, in shaft furnaces the residence time of material in the small melt pool at the base of the furnace is short, and the raw materials must be incorporated sufficiently rapidly in this pool of melt if a melt is to be obtained which is suitable for provision of final product having specified properties.

In the manufacture of mineral wool products, the fibres obtained in the spinning process are blown into a collection chamber and, while airborne and while still hot, are sprayed with a binder solution and randomly deposited as a mat or web onto a travelling conveyor. The fibre web or mat is then transferred to a curing oven where heated air is blown through the mat to cure the binder. The cured mat or slab is trimmed at the sides and cut up into certain dimensions. Both during spinning and during trimming, cutting up into final dimension and subsequent final inspection and check for defects, waste products are arising which are either dumped or, preferably, recycled to the MMVF production process.

To that end, the waste products are broken up into smaller, fine-grained pieces by milling in a rod mill or any appropriate device/equipment and/or unraveled and then compacted to form briquettes. Briquettes from MMVF waste are normally produced by moulding a mix of the MMVF waste, optionally together with other fine-grained components in finely divided form, and an appropriate binder into the desired briquette shape and curing the binder. Preferably, a cement binder is used to produce cement briquettes.

The briquettes, possibly after interim storage, may be combined with virgin raw material and/or other lump raw material such as slag for MMVF production and returned via the melting furnace into the MMVF production process. Briquettes are particularly useful for forming part, often most of the charge in a shaft or cupola furnace. The amount of briquettes may be up to 100%, such as up to 80% or 50%, of the total charge. They may also be used as part of the charge in an electric furnace.

When using MMVF waste for briquette production, the waste products may contain cured and/or uncured mineral wool binder, depending on the point in the production line where the waste products are formed. The mineral wool binder resins may, for instance, be conventional phenol/formaldehyde resins, optionally extended with urea, or formaldehyde-free binders such as, for instance, the binder compositions based on polycarboxy polymers and polyols or polyamines, such as disclosed in EP-A-583086, EP-A-990727 , EP-A-1741726 , US-A-5,318,990 and US-A-2007/0173588.

Another group of non-phenol/formaldehyde binders are the addition/elimination reaction products of aliphatic and/or aromatic anhydrides with alkanolamines, e.g., as disclosed in WO 99/36368, WO 01/05725, WO 01/96460, WO 02/06178, WO 2004/007615 and WO 2006/061249.

A further group of formaldehyde-free mineral wool binders are those which contain carbohydrates, for instance, sugar, as additives, extenders or as reactive components of the binder system; see, e.g., WO 2007/014236.

It has however been found that the presence of non-cured or partly cured sugar-containing mineral wool binder in the MMVF waste results in intolerable curing times of cement-containing briquettes, the reason being that sugar is a retarder for cement. The term sugar, as used herein, refers to carbohydrates such as monosaccharides, disaccharides, polysaccharides and mixtures thereof.

In order to overcome this problem, attempts have been made to replace cement in the production of the compacted bodies by starch. However, these attempts have proven unsatisfactory, in particular because it has been found that the water needed for the curing of the starch results in mold formation.

Summary of the Invention

Accordingly, it was an object of the present invention to provide compacted bodies, in particular briquettes, suitable for us as a mineral charge in the production of MMV fibres which, despite the presence of sugar-containing mineral wool binder in the MMVF waste, show satisfactory strength development during briquette production and at the same time not having the problem of mold formation during curing.

It was a further object of the present invention to provide a method for producing such a compacted body and the use of such a compacted body as a mineral charge in the production of MMV fibres of wool.

In accordance with a first aspect of the present invention, there is provided a compacted body, in particular a briquette, suitable for use as mineral charge in the production of man-made vitreous fibres (MMVF), said compacted body comprising:

(i) recycled mineral wool waste which comprises MMV fibres and a sugar component and
(ii) a binder comprising

- at least one phenol compound as described in claim 1,
- at least one protein.

In accordance with a second aspect of the present invention, there is provided a method of producing a compacted body, in particular a briquette, suitable for use as mineral charge in the production of man-made vitreous fibres (MMVF), said method comprising the steps of mixing

recycled mineral wool waste which comprises MMV fibres in contact with a non-cured MMVF binder comprising a sugar component and

a binder comprising

- at least one phenol compound as described in claim 1,

- at least one protein;

and curing the mixture to form said compacted body.

In accordance with a third aspect of the present invention, there is provided a use of a compacted body according to any one of claims 1 to 17 as a mineral charge in the production of MMV fibres or wool.

The present inventors have surprisingly found, that it is possible to provide a compacted body, in particular a briquette, suitable for use as a mineral charge in the production of man-made vitreous fibres (MMVF) in which the cement is replaced by a binder comprising at least one phenol and/or quinone-containing compound, and at least one protein.

Such a binder does not result in a retarding of the curing of the compacted body in the presence of a sugar component. Further, such a binder does not result in any molding problems. As an additional benefit, the sulfur content of such a compacted body is lowered drastically in the waste briquettes compared to using cement as a binder. This low sulfur-content has a beneficial environmental impact when using the waste briquettes in the production of mineral wool where the briquettes are melted and off-gassing occurs with a much lower sulfuroxides content. Accordingly, high environmental standards can be fulfilled when using the compacted body according to the present invention without the need for expensive gas-detoxification equipment which leads to economic advantages.

Description of the Preferred Embodiments

The compacted body, in particular briquette, suitable for use as a mineral charge for the production of man-made vitreous fibres (MMVF) according to the present invention comprises:

(i) recycled mineral wool waste which comprises MMV fibres and a sugar component and
(ii) a binder comprising

- at least one phenol containing compound,
- at least one protein.

## The Binder

In a preferred embodiment, the binders used in the compacted body according to the present invention are formaldehyde free.

For the purpose of the present application, the term "formaldehyde free" is defined to characterize a mineral wool product where the emission is below 5 $\mu$g/m$^2$/h of formaldehyde from the mineral wool product, preferably below 3 $\mu$g/m$^2$/h. Preferably, the test is carried out in accordance with ISO 16000 for testing aldehyde emissions.

### Phenol and/or quinone containing compound component of the binder

The binder composition used in the compacted body according to the present invention comprises a phenol and/or quinone containing compound component of the binder, in particular one or more phenolic compound as described in claim 1.

Phenolic compounds, or phenolics, are compounds that have one or more hydroxyl group attached directly to an aromatic ring. Polyphenols (or polyhydroxyphenols) are compounds that have more than one phenolic hydroxyl group attached to one or more aromatic rings. Phenolic compounds are characteristic of plants and as a group they are usually found as esters or glycosides rather than as free compounds.

The term phenolics covers a very large and diverse group of chemical compounds. Preferably, the phenol containing compound is a compound according to the scheme based on the number of carbons in the molecule as detailed in by W. Vermerris, R. Nicholson, in Phenolic Compound Biochemistry, Springer Netherlands, 2008.

The phenolic compounds in the frame of the invention are tannins, which comprise a group of compounds with a wide diversity in structure that share their ability to bind and precipitate proteins. Tannins are abundant in many different plant species, in particular oak, chestnut, staghorn sumac and fringe cups. Tannins can be present in the leaves, bark and fruits. Tannins can be classified into three groups: condensed tannins, hydrolysable tannins and complex tannins. Condensed tannins, or proanthocyanidins, are oligomeric or polymeric flavonoids consisting of flavan-3-ol (catechin) units. Gallotannins are hydrolysable tannins with a polyol core substituted with 10-12 gallic acid residues. The most commonly found polyol in gallotannins is D-glucose although some gallotannins contain catechin and triterpenoid units as the core polyol. Ellagitanins are hydrolysable tannins that differ from gallotannins in that they contain additional C-C bonds between adjacent galloyl moieties. Complex tannins are defined as tannins in which a catechin unit is bound glycosidically to either a gallotannin or an ellagitannin unit.

The inventors have surprisingly found that the tannins can be used to crosslink proteins which allows a binder composition to be formed. Often, these phenol containing compound components are obtained from vegetable tissues and are therefore a renewable material. In some embodiments, the compounds are also non-toxic and non-corrosive. As a further advantage, these compounds are antimicrobial and therefore impart their antimicrobial properties to the mineral wool product bound by such a binder.

In a preferred embodiment, the phenol containing compound is selected from one or more components from the group consisting of tannic acid, ellagitannins and gallotannins, tannin originating from one or more of oak, chestnut, staghorn sumac and fringe cups.

### Protein component of the binder

Preferably, the protein component of the binder is in form of one or more proteins selected from the group consisting of proteins from animal sources, including collagen, gelatine, hydrolysed gelatine, and protein from milk (casein, whey), eggs; proteins from vegetable sources, including proteins from legumes, cereals, whole grains, nuts, seeds and fruits, like protein from buckwheat, oats, rye, millet, maize (corn), rice, wheat, bulgar, sorghum, amaranth, quinoa, soybeans (soy protein), lentils, kidney beans, white beans, mung beans, chickpeas, cowpeas, lima beans, pigeon peas, lupines, wing beans, almonds, Brazil nuts, cashews, pecans, walnuts, cotton seeds, pumpkin seeds, hemp seeds, sesame seeds, and sunflower seeds; polyphenolic proteins such as mussel foot protein.

Collagen is a very abundant material in living tissue: It is the main component in connective tissue and constitutes 25-35% of the total protein content in mammals. Gelatin is derived from chemical degradation of collagen. Gelatin is water soluble and has a molecular weight of 30.000 to 300.000 g/mol dependent on the grade of hydrolysis. Gelatin is a widely used food product and it is therefore generally accepted that this compound is totally non-toxic and therefore no precautions are to be taken when handling gelatin.

The gelatin can also be further hydrolysed to smaller fragments of down to 3000 g/mol.

In a preferred embodiment, the protein component is gelatin, whereby the gelatin is preferably originating from one or more sources from the group consisting of mammal, bird species, such as from cow, pig, horse, fowl, and/or from scales, skin of fish.

Reaction of the binder components

Without wanting to be bound to any particular theory, the present inventors believe that the reaction between the phenol and/or quinone containing compound and the protein at least partly relies on a oxidation of phenols to quinones followed by nucleophilic attack of amine and/or thiol groups from the protein which leads to a crosslinking of the proteins by the phenol and/or quinone containing compounds.

The present inventors have found that the curing of the binder is strongly accelerated under alkaline conditions. Therefore, in one embodiment, the binder composition for mineral fibres comprises a pH-adjuster, preferably in form of a base, such as organic base, such as amine or salts thereof, inorganic bases, such as metal hydroxide, such as KOH or NaOH, ammonia or salts thereof.

In a particular preferred embodiment, the pH adjuster is an alkaline metal hydroxide, in particular NaOH.

In a preferred embodiment, the binder composition according to the present invention has a pH of 7 to 10, such as 7.5 to 9.5, such as 8 to 9.

In one embodiment, the protein comprises polyphenolic proteins.

These proteins contain a high level of a post-translationally modified-oxidized-form of tyrosine, L-3,4-dihydroxyphenylalanine (levodopa, L-DOPA). See also J. J. Wilker Nature Chem. Biol. 2011, 7, 579-580 for a reference to these proteins.

Additives

In a preferred embodiment, the binder used in the compacted body according to the present invention contains additives.

As already described above, many phenol and/or quinone containing compounds, in particular polyphenols, have antimicrobial properties and therefore impart antimicrobial characteristic to the binder. Nevertheless, in one embodiment, an anti-fouling agent may be added to the binder compositions.

In one embodiment, an anti-swelling agent may be added to the binder, such as tannic acid and/or tannins.

In one embodiment, the binder composition according to the present invention contains additives in form of amine linkers and/or thiol/thiolate linkers. These additives in form of amine linkers and/or thiol/thiolate linkers are particular useful when the crosslinking reaction of the binder proceeds via the quinone-amine and/or quinone-thiol pathway.

In one embodiment, the binder used in the compacted body according to the present invention comprises an additive containing metal ions, such as iron ions.

Polyphenolic proteins such as the mussel adhesive protein discussed above relies on 3,4-dihydroxyphenyl moieties to enhance the surface adhesion. This is achieved in combination with the secretion of selected types of cations such as iron ions. In one embodiment, the binder could be said to mimic the polyphenolic protein and therefore the addition of various cations could improve the binder characteristics. Such advantageous ions can also be released from the mineral fibre surface when they come into contact with the aqueous binder.

In one embodiment, the recycled mineral wool waste comprises stone wool. Without being bound by theory, it is believed that leaching of certain ions from the vitreous fibres may assist the binding strength. The mechanism may be analogue to the mechanism for which mussel adhesive protein obtains a surface adhesion. This is achieved in combination with the secretion of selected types of cations such as iron ions.

Oxidising agents as additives can serve to increase the oxidising rate of the phenolics. One example is the enzyme tyrosinase which oxidizes phenols to hydroxyphenols/quinones and therefore accelerates the binder forming reaction.

In another embodiment, the oxidising agent is oxygen, which is supplied to the binder.

In one embodiment, the curing is performed in oxygen-enriched surroundings.

**The Compacted Body**

In one embodiment, the compacted body according to the present invention further comprises cement, such as Portland Cement.

In this embodiment, only part of the cement conventionally used for the production of the compacted body is replaced by the binder described above. It has been found, that the presence of the binder described above leads to an acceleration of the curing of the cement. Without being bound to any particular theory, the present inventors believe that this is due to the presence of proteins in the binder. In a preferred embodiment, the binder used in the compacted bodies according to the present invention is applied as an aqueous binder. Since cement cures in the presence of water, the combination of the binder described above and cement is particularly beneficial.

In a preferred embodiment, the mineral material used in the compacted body according to the present invention is 100% mineral wool waste. Preferably, the compacted body according to the present invention may also contain other mineral material than mineral wool waste, such as raw materials for melting, such as alumina containing raw materials.

In a preferred embodiment, the sugar component in the recycled mineral wool waste is selected from sucrose and reducing sugars such as hexoses and pentoses, and mixtures thereof.

Preferably, the sugar component is a reducing sugar having a dextrose equivalent (DE) of 40 to 100, preferably 50 to 100, and more preferably 85 to 100.

In a particularly preferred embodiment, the sugar component is a reducing sugar selected from high DE glucose syrup and high-fructose syrup and mixtures thereof.

The present inventors have found that particularly good results can be obtained when the compacted bodies comprise 0.5 to 10 percent by weight, preferably 1 to 5 percent by weight, of cement, based on the total weight (dry matter) of the compacted body.

The present inventors have found that particularly good results can be obtained when the compacted bodies according to the present invention comprise 10 to 95, such as 20 to 90, such as 30 to 80 percent by weight of recycled mineral wool waste, based on the total weight (dry matter) of the compacted body.

In a preferred embodiment, the compacted body comprises 2 to 20, such as 5 to 15 percent by weight of binder, based on the total weight (dry matter) of the compacted body.

In a preferred embodiment, the compacted body according to the present invention has a density within the range of from 1500 to 2200 kg/m³.

## Method for Producing the Compacted Body

In the present invention, the briquettes may be produced by any method known in the art. Generally, the production process comprises the steps of

- mixing recycled mineral wool waste which comprises MMV fibres in contact with a non-cured MMVF binder comprising a sugar component and
  a binder comprising at least one phenol and/or quinone containing compound, at least one protein; and
- curing the mixture to form said compacted body.

In addition to the MMVF waste and the cement binder, the briquettes may include other suitable virgin or waste mineral charge materials, for instance, raw materials such as soda; iron ore; boron-containing materials; phosphorus-containing materials such as apatite; dolomite, quartz sand; olivine sand; limestone; rutile; magnesite; magnetite; brucite, bauxite, kaolin, ilmenite, alumina-containing material such as bauxite and filter dusts from the calcination of bauxite and other processes involving heating and/or calcination of high alumina materials; slag from the metallurgical industry, especially steelmaking slag such as converter slag or electric arc furnace slag, and slag from the ferro-alloy industry such as ferro-chromium, ferro-manganese or ferro-silica slag; slag and residues from the primary production of aluminium such as spent aluminium pot lining or red mud; dried or wet sludge from the paper industry; sewage sludge; bleaching clay; residues from the incineration of household and industrial wastes, especially slag or filter ashes from the incineration of municipal solid wastes; glass waste, for instance, from the vitrification of other waste products; glass cullet; waste products from the mining industry, especially minestone from the excavation of coal; residues from the incineration of fossil fuel, especially from the combustion of coal at power plants; spent abrasive sand; spent moulding sand from iron and steel casing; waste sieving sand; glass-fibre reinforced plastic; and fines and breakage waste from the ceramic and brick industry.

In a currently preferred method, the production of briquettes involves the following steps:

Collecting of MMVF waste with non-cured binder
Spinning waste with varying contents of non-cured binder is collected and milled separately or in combination with cured wool waste or in combination with cured wool waste and fines from rocks and briquettes and wool waste coming back from the market. Milling is performed in rod mills or hammer mills or another appropriate device which are run with or without additional water. Milling is preferably carried out to a degree such that the milled mixture has a density of e.g. 900 to 1500 kg/m³.

Mixing with aqueous binder and optionally cement

In the next step, the raw materials (including the MMVF waste) are added to a mixer and then the aqueous binder and optionally cement is added.

Compacting/shaping of the briquette mixture

The mixture is poured into moulds and pressed discontinuously under a pressure of, for instance, 25 to 60 kPa. The shape is not limited but a compact shape is preferred to avoid misalignment in the shaft oven. Currently preferred is the production of hexagon-shaped briquettes having a diameter of about 5 to 15 cm and a height of about 5 to 13 cm.

Curing

Curing of the briquettes is preferably effected at a curing temperature of from 15 to 65 °C, more preferably 30 to 40 °C. Curing times generally range from 2 h to 108 h, such as 12 h to 96 h.

Use of the briquettes as mineral charge in the production of MMV fibres

The briquettes produced in accordance with the invention are useful as mineral charge in any type of furnace which can be used to melt raw materials for making MMV fibres. The invention is particularly useful in shaft furnaces, especially in cupola furnaces.

The invention is especially beneficial in processes where a significant part (e.g. > 10%) of the charge is in the form of briquettes. Generally at least 20 to 25%, preferably at least 30% of the charge (by weight) is provided by briquettes. In some processes higher amounts, e. g. 45 to 55%, are preferred and amounts above 75% or even above 80% up to 100% are sometimes preferred. The remaining charge may be any suitable virgin or waste material.

The MMV fibres may be made from the fibre-forming mineral melt in conventional manner. Generally, they are made by a centrifugal fibre-forming process. For instance, the fibres may be formed by a spinning cup process in which they are thrown outwardly through perforations in a spinning cup, or melt may be thrown off a rotating disc and fibre formation may be promoted by blasting jets of gas through the melt. Preferably a cascade spinner is used and fibre formation is conducted by pouring the melt onto the first rotor in a cascade spinner. Preferably the melt is poured onto the first of a set of two, three or four rotors, each of which rotates about a substantially horizontal axis, whereby melt on the first rotor is primarily thrown onto the second (lower) rotor although some may be thrown off the first rotor as fibres, and melt on the second rotor is thrown off as fibres although some may be thrown towards the third (lower) rotor, and so forth.

The MMV fibres may be used for any of the purposes for which MMVF products are known. These include fire insulation and protection, thermal insulation, noise reduction and regulation, construction, horticultural media, and reinforcement of other products such as plastics and as a filler. The materials may be in the form of bonded batts or the materials may be comminuted into a granulate. Bonded batts include materials such as slabs and pipe sections.

Advantages of the compacted bodies according to the present invention

The compacted bodies according to the present invention have the advantage that they can be produced with a very simple binder which requires as little as only two components, namely at least one protein, at least one phenol and/or quinone-containing compound. The compacted bodies are therefore produced from natural and non-toxic components and are therefore safe to work with. At the same time, the compacted bodies according to the present invention are produced from a binder based on renewable sources.

A further advantage is the possibility of curing the compacted bodies at ambient temperature or in the vicinity of ambient temperature. This not only leads to savings of energy consumption but also enables the production of the compacted bodies with less complex machinery.

Further, the sulfur content of the compacted bodies according to the present invention is drastically reduced compared to compacted bodies prepared by using cement which leads to ecological and economic advantages.

**Example**

In the the following example, several compacted bodies which fall under the definition of the present invention were prepared and tested:

Binder used for the production of the compacted bodies

The following properties were determined for the binders according the present invention.

*Reagents*

Medium gel strength gelatin from porcine skin (170-195 g Bloom), tannic acid and sodium hydroxide were obtained from Sigma-Aldrich. For simplicity, these reagents were considered completely pure and anhydrous.

*Binder component solids content - definition*

The content of each of the components in a given binder solution before curing is based on the anhydrous mass of the components. The following formula can be used:

$$Binder\ component\ solids\ content\ (\%) = \frac{binder\ component\ A\ solids\ (g) + binder\ component\ B\ solids\ (g) + \cdots}{total\ weight\ of\ mixture\ (g)} \times 100\%$$

| | Gelatin, tannic acid, sodium hydroxide |
|---|---|
| **Example** | |
| **Binder composition** | |
| Protein (%-wt.) | |
| Gelatin (porcine skin), medium gel strength | 100 |
| Phenol and/or quinone containing compound (%-wt.) [a] | |
| Tannic acid | 2 |
| Base (%-wt.) [b] | |
| Sodium hydroxide | 1.7 |
| | |
| **Binder properties** | |
| Binder component solids content (%) | 14.6 |
| pH of binder mixture | 8.8 |
| | |
| **Curing conditions** | |
| Temperature (°C) | 35°C |

Briquette production

80g rod mill waste were mixed with 16, 18, 20, 22, and 24 g, respectively, of the binder described above.
The mixture was pressed hard together in a form.
The mixture was allowed to set for four hours at room temperature and was then dried for three days at 35°C.
All mixtures gave sufficiently strong briquettes.
A comparison of the handling gave the result, that the mixture which is the easiest to work with and to be pressed into a desired shape is the mixture of 4:1 (80 g rod mill waste and 20 g of the binder).

**Claims**

1. A compacted body, in particular a briquette, suitable for use as mineral charge in the production of man-made vitreous fibres (MMVF), said compacted body comprising:

(i) recycled mineral wool waste which comprises MMV fibres and a sugar component and
(ii) a binder comprising

- at least one tannins, such as condensed tannins (proanthocyanidins), such as hydrolysable tannins, such as gallotannins, such as ellagitannins, such as complex tannins, such as tannic acid,
- at least one protein.

2. A compacted body according to claim 1, wherein the tannin is selected from one or more components from the group consisting of tannic acid, condensed tannins (proanthocyanidins), hydrolysable tannins, gallotannins, ellagitannins, complex tannins, and/or tannin originating from one or more of oak, chestnut, staghorn sumac and fringe cups.

3. A compacted body according to any of the preceding claims, wherein the at least one protein is selected from the group consisting of proteins from animal sources, including collagen, gelatine, hydrolysed gelatine, and protein from milk (casein, whey), eggs; proteins from vegetable sources, including proteins from legumes, cereals, whole grains, nuts, seeds and fruits, like protein from buckwheat, oats, rye, millet, maize (corn), rice, wheat, bulgar, sorghum, amaranth, quinoa, soybeans (soy protein), lentils, kidney beans, white beans, mung beans, chickpeas, cowpeas, lima beans, pigeon peas, lupines, wing beans, almonds, Brazil nuts, cashews, pecans, walnuts, cotton seeds, pumpkin seeds, hemp seeds, sesame seeds, and sunflower seeds; polyphenolic proteins such as mussel foot protein.

4. A compacted body according to claim 3, wherein the collagen or gelatin is originating from one or more sources from the group consisting of mammal, bird species, such as from cow, pig, horse, fowl, and/or from scales, skin of fish.

5. A compacted body according to any of the preceding claims, wherein the binder composition further comprises an additive selected from the group of an oxidiser, such as tyrosinase, a metal ion, such as iron ion, a pH-adjuster, preferably in form of a base, such as organic base, such as amine or salts thereof, inorganic bases, such as metal hydroxide, such as KOH or NaOH, ammonia or salts thereof.

6. A compacted body according to any of the preceding claims, wherein the binder composition has a pH of more than 7, such as more than 8, such as more than 9.

7. A compacted body according to any of the preceding claims, wherein the MMV fibres are selected from stone wool and glass wool.

8. The compacted body according to any of the preceding claims, further comprising cement, such as Portland cement.

9. The compacted body of any of the preceding claims, wherein the sugar component is selected from sucrose and reducing sugars such as hexoses and pentoses, and mixtures thereof.

10. The compacted body of claim 9, wherein the sugar component is a reducing sugar having a dextrose equivalent (DE) of 40 to 100, preferably 50 to 100, and more preferably 85 to 100.

11. The compacted body of claim 8 or 9, wherein the sugar component is a reducing sugar selected from high DE glucose syrup and high-fructose syrup and mixtures thereof.

12. The compacted body of any of the preceding claims, which comprises 0.5 to 10 percent by weight, preferably 1 to 5 percent by weight, of cement, based on the total weight (dry matter) of the compacted body.

13. The compacted body of any of the preceding claims, which comprises 10 to 95, such as 20 to 90, such as 30 to 80 percent by weight of recycled mineral wool waste, based on the total weight (dry matter) of the compacted body.

14. The compacted body of any of the preceding claims, which has a density within the range of from 1500 to 2200 kg/m$^3$.

15. A method of producing a compacted body, in particular a briquette, suitable for use as mineral charge in the production of man-made vitreous fibres (MMVF), said method comprising the steps of

- mixing recycled mineral wool waste which comprises MMV fibres in contact with a non-cured MMVF binder comprising a sugar component and a binder comprising at least one tannins, such as condensed tannins (proanthocyanidins), such as hydrolysable tannins, such as gallotannins, such as ellagitannins, such as complex tannins, such as tannic acid, at least one protein; and
- curing the mixture to form said compacted body.

**16.** The method of claim 15, wherein curing of the mixture is effected at a curing temperature of from 15 to 65°C, preferably 30 to 40°C and at a relative humidity of > 70%, preferably > 90%.

**17.** The use of a compacted body according to any one of claims 1 to 14 as a mineral charge in the production of MMV fibres or wool.

**Patentansprüche**

**1.** Kompaktierter Körper, insbesondere ein Brikett, geeignet zur Verwendung als Mineralcharge bei der Herstellung von künstlichen glasartigen Fasern (MMVF), wobei der kompaktierte Körper:

(i) recycelten Mineralwolle-Abfall, der MMV-Fasern und eine Zuckerkomponente umfasst, und
(ii) ein Bindemittel, umfassend

- mindestens ein Tannin, wie z.B. kondensierte Tannine (Proanthocyanidine), wie z.B. hydrolysierbare Tannine, wie z.B. Gallotannine, wie z.B. Ellagitannine, wie z.B. komplexe Tannine, wie z.B. Gerbsäure,
- mindestens ein Protein,

umfasst.

**2.** Kompaktierter Körper nach Anspruch 1, wobei das Tannin ausgewählt ist aus einer oder mehreren Komponenten aus der Gruppe bestehend aus Gerbsäure, kondensierten Tanninen (Proanthocyanidinen), hydrolysierbaren Tanninen, Gallotanninen, Ellagitanninen, komplexen Tanninen und/oder Tannin, das von einem oder mehreren aus Eiche, Kastanie, Essigbaum und Fransenbecher stammt.

**3.** Kompaktierter Körper nach irgendeinem der vorstehenden Ansprüche, wobei das mindestens eine Protein ausgewählt ist aus der Gruppe bestehend aus Proteinen aus tierischen Quellen, einschließlich Kollagen, Gelatine, hydrolysierter Gelatine und Protein aus Milch (Kasein, Molke), Eiern; Proteinen aus pflanzlichen Quellen, einschließlich Proteinen aus Hülsenfrüchten, Getreiden, Vollkorngetreiden, Nüssen, Samen und Früchten, wie z.B. Protein aus Buchweizen, Hafer, Roggen, Hirse, Mais (Korn), Reis, Weizen, Bulgar, Sorghum, Amaranth, Quinoa, Sojabohnen (Sojaprotein), Linsen, Kidneybohnen, weißen Bohnen, Mungbohnen, Kichererbsen, Kuherbsen, Limabohnen, Straucherbsen, Lupinen, Goabohnen, Mandeln, Paranüssen, Cashewnüsse, Pekannüssen, Walnüssen, Baumwollsamen, Kürbiskernen, Hanfsamen, Sesamsamen und Sonnenblumenkernen; polyphenolischen Proteinen, wie z.B. Muschelfußprotein.

**4.** Kompaktierter Körper nach Anspruch 3, wobei das Kollagen oder die Gelatine aus einer oder mehreren Quellen aus der Gruppe bestehend aus Säugetier, Vogelarten, wie z.B. von Rind, Schwein, Pferd, Geflügel, und/oder aus Schuppen, Haut von Fischen stammt.

**5.** Kompaktierter Körper nach irgendeinem der vorstehenden Ansprüche, wobei die Bindemittelzusammensetzung ferner ein Additiv umfasst, das aus der Gruppe eines Oxidationsmittels, wie z.B. Tyrosinase, eines Metallions, wie z.B. Eisenion, eines pH-Einstellmittels, bevorzugt in Form einer Base, wie z.B. organische Base, wie z.B. Amin oder Salze davon, anorganischen Basen, wie z.B. Metallhydroxid, wie z.B. KOH oder NaOH, Ammoniak oder Salze davon, ausgewählt ist.

**6.** Kompaktierter Körper nach irgendeinem der vorstehenden Ansprüche, wobei die Bindemittelzusammensetzung einen pH-Wert von mehr als 7, wie z.B. mehr als 8, wie z.B. mehr als 9, aufweist.

**7.** Kompaktierter Körper nach irgendeinem der vorstehenden Ansprüche, wobei die MMV-Fasern aus Steinwolle und Glaswolle ausgewählt sind.

**8.** Kompaktierter Körper nach irgendeinem der vorstehenden Ansprüche, der ferner Zement, wie z.B. Portlandzement, umfasst.

**9.** Kompaktierter Körper nach irgendeinem der vorstehenden Ansprüche, wobei die Zuckerkomponente ausgewählt ist aus Saccharose und reduzierenden Zuckern, wie z.B. Hexosen und Pentosen, und Mischungen davon.

**10.** Kompaktierter Körper nach Anspruch 9, wobei die Zuckerkomponente ein reduzierender Zucker mit einem Dextrose-Äquivalent (DE) von 40 bis 100, bevorzugt 50 bis 100 und bevorzugter 85 bis 100 ist.

**11.** Kompaktierter Körper nach Anspruch 8 oder 9, wobei die Zuckerkomponente ein reduzierender Zucker ist, ausgewählt aus Glucosesirup mit hohem DE-Gehalt und Sirup mit hohem Fructosegehalt und Mischungen davon.

**12.** Kompaktierter Körper nach irgendeinem der vorstehenden Ansprüche, der 0,5 bis 10 Gewichtsprozent, bevorzugt 1 bis 5 Gewichtsprozent, Zement, bezogen auf das Gesamtgewicht (Trockenmasse) des kompaktierten Körpers, umfasst.

**13.** Kompaktierter Körper nach irgendeinem der vorstehenden Ansprüche, der 10 bis 95, wie z.B. 20 bis 90, wie z.B. 30 bis 80 Gewichtsprozent, recycelten Mineralwolle-Abfall, bezogen auf das Gesamtgewicht (Trockenmasse) des kompaktierten Körpers, umfasst.

**14.** Kompaktierter Körper nach irgendeinem der vorstehenden Ansprüche, der eine Dichte im Bereich von 1500 bis 2200 kg/m$^3$ aufweist.

**15.** Verfahren zur Herstellung eines kompaktierten Körpers, insbesondere eines Briketts, der zur Verwendung als Mineralcharge bei der Herstellung von künstlichen glasartigen Fasern (MMVF) geeignet ist, wobei das Verfahren die folgenden Schritte umfasst:

- Mischen von recyceltem Mineralwolle-Abfall, umfassend MMV-Fasern in Kontakt mit einem nicht gehärteten MMVF-Bindemittel, umfassend eine Zuckerkomponente, und einem Bindemittel, das mindestens ein Tannin umfasst, wie z.B. kondensierte Tannine (Proanthocyanidine), wie z.B. hydrolysierbare Tannine, wie z.B. Gallotannine, wie z.B. Ellagitannine, wie z.B. komplexe Tannine, wie z.B. Gerbsäure, mindestens ein Protein; und
- Härten der Mischung, um den kompaktierten Körper zu bilden.

**16.** Verfahren nach Anspruch 15, wobei das Härten der Mischung bei einer Härtungstemperatur von 15 bis 65°C, bevorzugt 30 bis 40°C und bei einer relativen Feuchtigkeit von > 70%, bevorzugt > 90%, erfolgt.

**17.** Verwendung eines kompaktierten Körpers nach irgendeinem der Ansprüche 1 bis 14 als eine Mineralcharge bei der Herstellung von MMV-Fasern oder -Wolle.

## Revendications

**1.** Corps compacté, en particulier une briquette, approprié pour une utilisation comme charge minérale dans la production de fibres vitreuses artificielles (MMVF), ledit corps compacté comprenant :

(i) un déchet recyclé de laine minérale qui comprend des fibres MMV et un composant sucre et
(ii) un liant comprenant

- au moins un tanin, tel que des tanins condensés (proanthocyanidines), tels que des tanins hydrolysables, tels que des gallotanins, tels que des ellagitanins, tels que des tanins complexes, tels que l'acide tannique,
- au moins une protéine.

**2.** Corps compacté selon la revendication 1, dans lequel le tanin est choisi parmi un ou plusieurs composants du groupe constitué par l'acide tannique, les tanins condensés (proanthocyanidines), les tanins hydrolysables, les gallotanins, les ellagitanins, les tanins complexes, et/ou un tanin provenant d'un ou plusieurs parmi le chêne, le châtaignier, le sumac vinaigrier et la tellima grandiflora.

**3.** Corps compacté selon l'une quelconque des revendications précédentes, dans lequel l'au moins une protéine est choisie dans le groupe constitué par les protéines de sources animales, y compris le collagène, la gélatine, la gélatine hydrolysée et une protéine du lait (caséine, lactosérum), les œufs ; les protéines de sources végétales, y compris les protéines de légumineuses, de céréales, de céréales complètes, de noix, de graines et de fruits, comme les protéines de sarrasin, d'avoine, de seigle, de millet, de maïs, de riz, de blé, de boulgour, de sorgho, d'amarante, de quinoa, de germes de soja (protéine de soja), de lentilles, de haricots rouges, de haricots blancs, de haricots mungo, de pois chiches, de niébés, de haricots de Lima, de pois d'Angole, de lupins, de haricots ailés, d'amandes, de noix du Brésil, de noix de cajou, de noix de pécan, de noix, de graines de coton, de graines de citrouille, de graines de chanvre, de graines de sésame et de graines de tournesol ; les protéines polyphénoliques telles que la protéine de pied de moule.

**4.** Corps compacté selon la revendication 3, dans lequel le collagène ou la gélatine provient d'une ou plusieurs sources du groupe constitué par les mammifères, les espèces aviaires, tels que des vaches, des cochons, des chevaux, de la volaille, et/ou d'écaillés, de peau de poisson.

**5.** Corps compacté selon l'une quelconque des revendications précédentes, dans lequel la composition de liant comprend en outre un additif choisi dans le groupe constitué par un oxydant, tel que la tyrosinase, un ion métallique, tel que l'ion fer, un ajusteur de pH, de préférence sous la forme d'une base, telle qu'une base organique, telle qu'une amine ou des sels de celle-ci, des bases inorganiques, telles que l'hydroxyde métallique, tel que KOH ou NaOH, l'ammoniac ou les sels de ceux-ci.

**6.** Corps compacté selon l'une quelconque des revendications précédentes, dans lequel la composition de liant a un pH supérieur à 7, tel que supérieur à 8, tel que supérieur à 9.

**7.** Corps compacté selon l'une quelconque des revendications précédentes, dans lequel les fibres MMV sont choisies parmi la laine de roche et la laine de verre.

**8.** Corps compacté selon l'une quelconque des revendications précédentes, comprenant en outre du ciment, tel que le ciment Portland.

**9.** Corps compacté selon l'une quelconque des revendications précédentes, dans lequel le composant sucre est choisi parmi le saccharose et les sucres réducteurs tels que les hexoses et les pentoses, et des mélanges de ceux-ci.

**10.** Corps compacté selon la revendication 9, dans lequel le composant sucre est un sucre réducteur ayant un équivalent de dextrose (DE) de 40 à 100, de préférence de 50 à 100, et plus préférablement de 85 à 100.

**11.** Corps compacté selon la revendication 8 ou 9, dans lequel le composant sucre est un sucre réducteur choisi parmi un sirop de glucose à DE élevé et un sirop riche en fructose et des mélanges de ceux-ci.

**12.** Corps compacté selon l'une quelconque des revendications précédentes, qui comprend de 0,5 à 10 % en poids, de préférence de 1 à 5 % en poids, de ciment, sur la base du poids total (matière sèche) du corps compacté.

**13.** Corps compacté selon l'une quelconque des revendications précédentes, qui comprend de 10 à 95, tel que de 20 à 90, tel que de 30 à 80 % en poids de déchet recyclé de laine minérale, sur la base du poids total (matière sèche) du corps compacté.

**14.** Corps compacté selon l'une quelconque des revendications précédentes, qui a une densité comprise dans la plage allant de 1 500 à 2 200 kg/m$^3$.

**15.** Procédé de production d'un corps compacté, en particulier d'une briquette, approprié pour une utilisation comme charge minérale dans la production de fibres vitreuses artificielles (MMVF), ledit procédé comprenant les étapes consistant à

- mélanger un déchet recyclé de laine minérale qui comprend des fibres MMV en contact avec un liant de MMVF non durci comprenant un composant sucre et un liant comprenant au moins un tanin, tel que des tanins condensés (proanthocyanidines), tels que des tanins hydrolysables, tels que des gallotanins, tels que des ellagitanins, tels que des tanins complexes, tels que l'acide tannique, au moins une protéine ; et
- durcir le mélange pour former ledit corps compacté.

**16.** Procédé selon la revendication 15, dans lequel le durcissement du mélange est effectué à une température de durcissement de 15 à 65°C, de préférence de 30 à 40°C et à une humidité relative > 70 %, de préférence > 90 %.

17. Utilisation d'un corps compacté selon l'une quelconque des revendications 1 à 14 en tant que charge minérale dans la production de fibres MMV ou de laine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 583086 A
- EP 990727 A
- EP 1741726 A
- US 5318990 A
- US 20070173588 A
- WO 9936368 A
- WO 0105725 A
- WO 0196460 A
- WO 0206178 A
- WO 2004007615 A
- WO 2006061249 A
- WO 2007014236 A

**Non-patent literature cited in the description**

- **W. VERMERRIS ; R. NICHOLSON.** Phenolic Compound Biochemistry. Springer, 2008
- **J. J. WILKER.** *Nature Chem. Biol.,* 2011, vol. 7, 579-580